# EUROPÄISCHE PATENTSCHRIFT

(11) **EP 1 787 598 B1**
(45) Veröffentlichungstag und Bekanntmachung des Hinweises auf die Patenterteilung: **28.04.2010**
(21) Anmeldenummer: 06023195.8
(22) Anmeldetag: 08.11.2006
(51) Int. Cl.: A61B 19/00, B08B 3/12, B08B 9/04

(54) **Verfahren und Vorrichtung zur Reinigung von Hohlgegenständen mittels Ultraschall**
Method and device for ultrasonically cleaning hollow bodies
Procédé et dispositif de nettoyage par ultrasons d'objets creux

(30) Priorität: 21.11.2005 DE 102005055291
(43) Veröffentlichungstag der Anmeldung: 23.05.2007
(73) Patentinhaber: Vanguard AG, 10117 Berlin (DE)
(72) Erfinder: Kahlen, Insa, 14059 Berlin (DE); Kraft, Mark Prof. Dr., 15366 Birkenstein (DE)
(74) Vertreter: Haft, von Puttkamer, Berngruber, Karakatsanis

(56) Entgegenhaltungen:
- EP-A1- 0 615 792
- EP-A2- 1 197 177
- CH-A5- 695 137
- DE-A1- 3 532 319
- DE-A1- 4 432 683
- DE-A1- 19 539 806
- DE-A1- 19 909 197
- US-A- 4 433 916

## Beschreibung

Die vorliegende Erfindung betrifft eine Vorrichtung und ein Verfahren zur Reinigung des Hohlraumes lumens eines medizinischen Gegenstandes.

Derzeit werden medizinische Instrumente in herkömmlichen Ultraschallbädern gereinigt. Dabei werden in einem Behälter, der mit einer Übertragungsflüssigkeit, beispielsweise Wasser, gefüllt ist, mit der Hilfe von sogenannten Sonotroden, die beispielsweise am Boden des Behälters angeordnet sind, Ultraschallwellen erzeugt, die sich im Wasser ausbreiten, das als Vorlaufstrecke dient. Die Frequenz der von den Sonotroden erzeugten Ultraschallwellen liegt in einem Bereich von 20 KHz bis 2 GHz. Diese Frequenz wird in Abhängigkeit von der kleinsten Struktur des zu reinigenden medizinischen Instrumentes ausgewählt. Die derzeit übliche Frequenz beträgt für Partikel mit einem Durchmesser größer 1 µm ca. 200 KHz. Bei 400 KHz bis 1 bis 2 MHz werden Partikel mit einem Durchmesser kleiner 1 µm optimal gereinigt. In der Übertragungsflüssigkeit entstehen insbesondere durch Interferenzen der Ultraschallwellen in an sich bekannter Weise Druckschwankungen in der Form von sich abwechselnden Über-und Unterdrücken, wobei beim Unterschreiten des Dampfdruckes der Übertragungsflüssigkeit Dampfbläschen erzeugt werden, die danach wieder kollabieren. Bei der Implosion der Dampfbläschen entstehen sogenannte Mikrojets in der Form von kleinen Wasserstrahlen, die sich bevorzugt in Richtung angrenzender Oberflächen ausdehnen, und an diesen hohe Drücke bewirken, die zu einer mechanischen Abtragung von Kontaminationen der Oberflächen der zu reinigenden Instrumente führen. Die Schall- bzw. Kavitationswirkung besteht ausschließlich in einer Abtragung bzw. Ablösung der Kontaminationen. Die Reinigung von Instrumenten erfordert zusätzlich einen Abtrag der Partikel durch Strömungen und gegebenenfalls die zusätzliche Unterstützung der Reinigung durch weitere physikalische oder chemische Vorgänge (z.B. unter Nutzung von Reinigungs- und Desinfektionsmitteln).

Eine nach diesem Prinzip arbeitende Ultraschallwaschmaschine geht aus der DE 39 24 519 A1 hervor, die u. a. auch für gewerbliche und industrielle Anwendungen geeignet ist.

Aus der DE 44 32 683 geht eine Vorrichtung zur äußeren und inneren Reinigung von kontaminierten Kathetern und Endoskopen mit Hilfe von Ultraschall hervor, wobei der Ultraschall über einen flexiblen Wellenleiter in die mit Flüssigkeiten gefüllten Arbeits- und Biopsiekanäle geleitet wird.

Aus der EP-A1-0 615 792 geht ein Verfahren zur Reinigung des Hohlraumes eines Gegenstandes hervor, wobei von wenigstens einem Ultraschallgenerator erzeugte Ultraschallwellen in das Lumen des Gegenstandes eingekoppelt werden. Dabei werden der Ultraschallgenerator und der Gegenstand in einem Behältnis mit einer Übertragungsflüssigkeit angeordnet.

In der EP-A2-1 197 177 ist ein Verfahren zur Reinigung des Lumens eines medizinischen Gegenstandes erläutert, bei dem ein Stabteil in das Lumen eingeschoben wird. Das Stabteil weist von seinem freien Ende beabstandete Ultraschallgeneratoren auf. Bei der Reinigung durchströmt eine Übertragungsflüssigkeit das Lumen des Gegenstandes. Ferner kann dabei aus einem das Stabteil umgebenden Rohr eine Reinigungsflüssigkeit abgegeben werden, die die durch Ultraschallwellen von der Innenfläche des Lumens abgetrennten Partikel aus dem Lumen austragen.

In der DE 199 09 197 A1 ist eine Vorrichtung zur Reinigung des Hohlraumes eines Lumens eines medizinischen Gegenstandes offenbart, die die Merkmale des Oberbegriffes des Anspruchs 1 aufweist.

Aus der DE 35 32 319 A1 geht ein gattungsfremdes Rohrreinigungsgerät zur Reinigung von unter den Kellerräumen von Häusern und Gebäuden verzeigt verlaufenden Abwasserkanälen hervor, das die Form eines konzentrischen Doppelrohres aufweist, wobei an dem vorderen Ende des Doppelrohres ein Düsenkopf mit nach hinten gestellten Düsen angeordnet ist.

Die Aufgabe der vorliegenden Erfindung besteht darin, eine Vorrichtung und ein Verfahren zur effektiven Reinigung des Hohlraumes eines Lumens eines medizinischen Gegenstandes zu schaffen.

Diese Aufgabe wird durch eine Vorrichtung mit den Merkmalen des Patentanspruches 1 sowie durch ein Verfahren mit den Merkmalen des Patentanspruches 5 gelöst.

Der wesentliche Vorteil der vorliegenden Erfindung besteht darin, dass durch sie eine effektive Reinigung von lumen, insbesondere von langgestreckten Lumen geringer Durchmesser von medizinischen Gegenständen ermöglicht wird.

Bei der vorliegenden Erfindung erfolgt die Reinigung derart, dass die Schallwellen eines externen Ultraschallgenerators über eine mechanische Schall-Kopplung in das Lumen des medizinischen Gegenstandes übertragen werden.

Bei bevorzugten Ausführungsform der vorliegenden Erfindung wird ein Miniatur-Ultraschallgenerator direkt in das Innenlumen von medizinischen Gegenständen eingebracht, wobei vorzugsweise eine zur Reinigung ausreichende Leistungsabgabe möglich ist.

Vorteilhafterweise kann die Schallfeldausbreitung- und - wirkung an definierte Hohlraumgeometrien angepasst werden.

Eine Unterstützung der durch die Ultraschallgeneratoren bewirkten Reinigung erfolgt vorteilhaft auch durch überlagerte Strömungen, die beispielsweise aus dem Hoheraum des Lumens des Gegenstandes austreten, wobei der Ultraschall der Ultraschallgeneratoren dann durch das strömende Medium selbst übertragen werden kann.

Weitere vorteilhafte Ausgestaltungen der Erfindung gehen aus den Unteransprüchen hervor.

Im folgenden werden die Erfindung und deren Ausgestaltungen im Zusammenhang mit den Figuren näher erläutert. Es zeigen:
Figur 1 in schematischer Darstellung eine Ausführungsform der erfindungsgemäßen Vorrichtung, bei der die von einer Ultraschallgenerator erzeugten Ultraschallschallwellen über eine mechanische Schall-Übertragung in das Lumen eines medizinischen Gegenstandes eingekoppelt werden;
Figur 2 eine Darstellung zur Erläuterung der Anpassung der erzeugten Schallenergie an die Reinigung einer definierten HohlraumGeometrie eines medizinischen Gegenstandes und
Figuren 3a und 3b die Ausgestaltung des Endbereiches des Stabteiles des erfindungsgemäßen Vorrichtung.
Figuren 5 bis 8a, 8b Weiterbildungen der Erfindung.

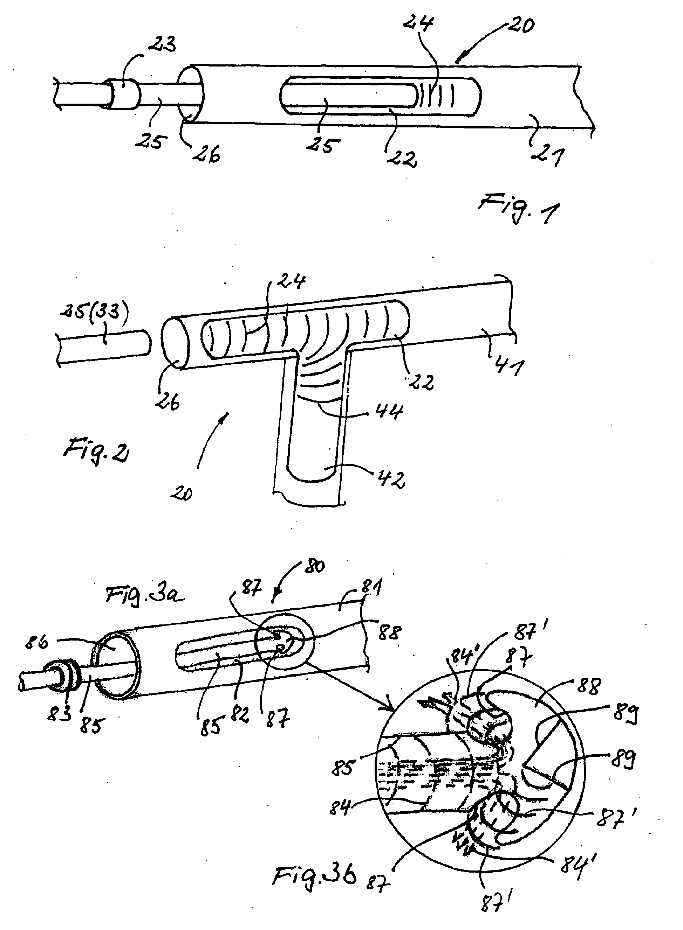

Im folgenden wird im Zusammenhang mit der Figur 1 eine Ausführungsform eines erfindungsgemäßen Vorrichtung 20 erläutert, bei der ein externer Ultraschallgenerator 23 mit einer mechanischen Vorrichtung, die die Form eines Stabteiles 25 aufweist, gekoppelt ist, wobei während der Reinigung des Ultraschallgenerator 23 außerhalb des Lumens 22 des in einem nicht dargestellten Behälter in einer Übertragungsflüssigkeit angeordneten Gegenstandes 21 verbleibt. Es wird lediglich das Stabteil 25 von außen durch eine Öffnung 26 des Gegenstandes 21 in das Lumen 22 eingeschoben, wobei die Ultraschallschallwellen von dem Ultrashallgenerator 23 auf das Stabteil 25 übertragen werden und sich von dessen Stirnfläche aus in der Übertragungsflüssigkeit des Lumens 22 ausbreiten (Bezugszeichen 24). Zur Verdeutlichung ist ein Teil des Gegenstandes 21 aufgebrochen dargestellt. Auf diese Weise kann die Reinigung der Innenwandung des Lumens 22 durch Einschieben des Stabteiles 25 erfolgen. Auf diese Weise können Lumen 22 von Gegenständen 21 gereinigt werden, deren Reinigung allein durch eine Strömungsenergie oder durch eine. Einwirkung von chemischen Substanzen nur schwer möglich ist.

Im Zusammenhang mit der Figur 2 wird die Reinigung einer speziellen Hohlraumgeometrie eines Lumens mit einer Vorrichtung 20 gemäß Figur 1 erläutert. Dabei wird das Ende des Stabteiles 25 der Vorrichtung 20 der Figur 1 bis in die Nähe der speziellen Hohlraumgeometrie, bei der es sich hier beispielsweise um eine T-förmige Abzweigung des Lumens 22 handelt, geschoben. Der abzweigende Bereich des Lumens 22 ist mit 42 bezeichnet. Ausgehend von dem Ende 22 des Stabteiles 25 breiten sich Ultraschallwellen 24 bis zur Abzweigstelle im Lumen 22 aus. Im Bereich der Abzweigstelle tritt ein Teil 44 der Ultraschallschallwellen in das abzweigende Lumen 42 ein.

Die Figuren 3a und 3b zeigen die Ausgestaltung des Endbereichen des Stabteiles 85 des vorliegenden erfindungsgemäßen Vorrichtung 80, bei der das Stabteil 85 die Form eines hohlen Rohres aufweist, das durch die Öffnung 86 in das Lumen 82 des Gegenstandes 81 eingeführt wird. An seinem freien Ende weist das hohle Stabteil 85 düsenartige Öffnungen 87 auf, die von seinem freien Ende weg weisen und zur Innenwand des Gegenstandes 81 gerichtet sind. Dadurch wird erreicht, dass eine in das hohle Stabteil 85 mit einem vorbestimmten Druck eingebrachte und durch die Ultraschallschwingungen der Sonotrode 83 beaufschlagte Übertragungsflüssigkeit aus den düsenartigen Öffnungen 87 entgegen der Vorschubrichtung des Stabteiles 85 in der Form von Strahlen 84' (Figur 3b) ausströmt und gegen die Innenwand des Gegenstandes 81 strömt, um diesen zu reinigen. Um die Ultraschallwellen der Übertragungsflüssigkeit in die genannte Richtung der Strahlen 84' der aus den Öffnungen 87 austretenden Übertragungsflüssigkeit zu lenken, sind in dem kopfteilartig ausgebildeten freien Ende 88 des Stabteiles 85 in der aus der vergrößerten Darstellung der Figur 5b ersichtlichen Weise Reflektorelemente 89 angeordnet. Die an den Reflektorelementen 89 reflektierten Ultraschallwellen sind mit 87' bezeichnet.

Es ist besonders vorteilhaft, wenn die aus den Öffnungen 87 ausströmende Übertragungsflüssigkeit entgegen der Vorschubrichtung des Stabteiles 85 abgesaugt wird, um bereits von der Innenwand des Gegenstandes 81 abgelöste Partikel auszuspülen.

Das an sich starr ausgebildete Stabteil 25, 85 kann auch aus starren Einzelteilen bestehen, die in seiner Längsrichtung an voneinander beabstandeten Orten gelenkig miteinander verbunden sind. Ferner kann das Stabteil der beschriebenen Ausführungsformen zur Erhöhung der Reinigungswirkung während des Reinigungsvorganges bewegt werden.

## Patentansprüche

1. Vorrichtung zur Reinigung des Hohlraumes eines Lumens (82) eines medizinischen Gegenstandes (81), wobei von wenigstens einem, an einem Stabteil (85) angeordneten Ultraschallgenerator (83) erzeugte Ultraschallschallwellen (84) in das Lumen (82) eingekoppelt werden, wobei der Ultraschallgenerator (83) und der Gegenstand (81) in einem Behältnis mit einer Übertragungsflüssigkeit angeordenbar sind, die auch in das Lumen (82) eintritt, wobei das Stabteil (85) von seinem Endbereich beabstandet wenigstens einen Ultraschallgenerator (83) aufweist und durch eine Öffnung (86) des Gegenstandes (81) in das Lumen (82) desselben einschiebbar ist, wobei die von dem Ultraschallgenerator (83) erzeugten Ultraschallschallwellen (84) auf das Stabteil (85) übertragen werden und sich vom freien Endbereich desselben in die im Lumen (82) befindliche Übertragungsflüssigkeit ausbreiten, wobei das Stabteil (85) rohrförmig ausgebildet und an seinem freien Ende verschlossen ist, **dadurch gekennzeichnet, dass** das stabteil (85) im Bereich seines freien Endes düsenartige Öffnungen (87) derart aufweist, dass eine in das Stabteil (85) eingebrachte Übertragungsflüssigkeit aus den Öffnungen (87) entgegen der Vorschubrichtung des Stabteiles (85) in der Form von Strahlen (84') der Übertragungsflüssigkeit zur Innenfläche des Lumens (82) ausströmt, dass die Strahlen (84') von den von dem Ultraschallgenerator (83) erzeugten Ultraschallwellen beaufschlagbar sind, und dass im freien Endbereich des Stabteiles (85) Reflektorelemente (89) vorgesehen sind, die sich in der Übertragungsflüssigkeit des rohrförmigen Stabteiles (85) zum freien Ende des Stabteiles (85) hin ausbreitende Ultraschallwellen (84) zu den Strahlen (84') ablenken.

2. Vorrichtung nach Anspruch 1, **dadurch gekennzeichnet, dass** als Ultraschallgenerator (83) ein Miniatur-Ultraschallgenerator verwendet ist.

3. Vorrichtung nach Anspruch 1 oder 2, **dadurch gekennzeichnet, dass** das Stabteil (85) starr ausgebildet ist.

4. Vorrichtung nach einem der Ansprüche 1 bis 3, **dadurch gekennzeichnet, dass** das Stabteil (85) aus starren Einzelteilen besteht, die in seiner Längsrichtung an voneinander beabstandeten Orten gelenkig miteinander verbunden sind.

5. Verfahren zur Reinigung des Hohlraumes eines Lumens (82) eines medizinischen Gegenstandes mit einer Vorrichtung nach einem der Ansprüche 1 bis 4, **dadurch gekennzeichnet, dass** in dem Lumen (82) des Gegenstandes (81) zusätzlich eine entgegen der Vorschubrichtung des Stabteiles (85) gerichtete Strömung der Übertragungsflüssigkeit erzeugt wird, um die durch die Strahlen (84') abgelösten Partikel aus dem Lumen (82) auszutragen.

6. Verfahren nach Ansprüchen 5 oder 6, **dadurch gekennzeichnet, dass** das Stabteil (85) während des Reinigungsvorganges bewegt wird.

## Claims

1. Apparatus for cleaning the cavity inside a lumen (82) of a medical article (81) in which ultrasonic waves (84) generated by at least one ultrasonic generator (83) disposed on a rod member (85) are coupled into lumen (82) and in which the ultrasonic generator (83) and the article (81) are adapted to be placed in a container holding a transmission fluid, said fluid also entering lumen (82), with rod member (85) having at least one ultrasonic generator (83) disposed at a distance from its end portion and being adapted to be inserted into lumen (82) of article (81) through an opening (86) in the latter, with the ultrasonic waves (84) generated by ultrasonic generator (83) being transmitted to rod member (85) and propagating from the free end portion thereof into the transmission fluid in lumen (82), and with rod member (85) being tubular and closed at one end, **characterized in that** rod member (85) has therein in the area of its free end nozzle-like apertures (87) such that transmission fluid introduced in rod member (85) exits from opening (87) in a direction opposite to the advance of rod member (85) and in the form of jets (84') of said transmission fluid directed towards the inner walls of lumen (82), **in that** jets (84') are acted upon by the ultrasonic waves generated by ultrasonic generator (83), and **in that** reflector elements (89) are provided in the free end area of rod member (85) to deflect towards jets (84') ultrasonic waves (84) which propagate in the transmission fluid inside tubular rod member (85) towards the free end thereof.

2. Apparatus as in claim 1, **characterized by** ultrasonic generator (83) comprising a miniature ultrasonic generator.

3. Apparatus as in claim 1 or 2, **characterized by** rod member (85) being rigid.

4. Apparatus as in any of claims 1 to 3, **characterized by** rod member (85) consisting of rigid parts articulated to each other at spaced locations in the longitudinal direction of rod member (85).

5. A method of cleaning the cavity inside a lumen (82) of a medical article by means of apparatus as in any of claims 1 to 4, **characterized by** additionally generating in lumen (82) of article (81) a flow of transmission fluid in a direction opposite to the advance of rod member (85), whereby the particles detached by jets (84') are flushed from lumen (82).

6. Method as in claim 5 or 6, **characterized by** rod member (85) being moved in the cleaning process.

## Revendications

1. Dispositif de nettoyage d'une cavité d'un lumen (82) d'un objet (81) médical, des ondes ultrasoniques (84) engendrées par au moins un générateur d'ultrasons (83) agencé sur une tige (85) étant injectées dans le lumen (82), le générateur d'ultrasons (83) et l'objet (81) pouvant être agencés dans un réceptacle avec un liquide de transmission qui pénètre aussi dans le lumen (82), la tige (85) présentant à distance de sa zone d'extrémité au moins un générateur d'ultrasons (83) et pouvant être introduite à travers une ouverture (86) de l'objet (81) dans le lumen (82) de celui-ci, les ondes ultrasoniques (84) engendrées par le générateur d'ultrasons (83) étant transmises à la tige (85) et se propageant depuis la zone d'extrémité libre de celle-ci dans le liquide de transmission se trouvant dans le lumen (82), la tige (85) étant réalisée en forme de tube et fermée à son extrémité libre, **caractérisé en ce que** la tige (85) présente dans la zone de son extrémité libre des ouvertures (87) en forme de buses de telle sorte qu'un liquide de transmission introduit dans la tige (85) s'écoule sous forme de jets (84') du liquide transmission hors des ouvertures (87) en sens contraire de la direction d'avancement de la tige (85) vers la surface intérieure du lumen (82), **en ce que** les jets (84') peuvent être sollicités par les ondes ultrasoniques engendrées par le générateur d'ultrasons (83), et **en ce que** dans la zone d'extrémité libre de la tige (85) sont prévus des éléments réflecteurs (89) qui dévient vers les jets (84') des ondes ultrasoniques (84) qui se propagent, dans le liquide de transmission de la tige (85) en forme de tube, vers l'extrémité libre de la tige (85).

2. Dispositif selon la revendication 1, **caractérisé en ce qu'**on utilise en tant que générateur d'ultrasons (83) un générateur d'ultrasons miniature.

3. Dispositif selon la revendication 1 ou 2, **caractérisé en ce que** la tige (85) est réalisée inflexible.

4. Dispositif selon l'une des revendications 1 à 3, **caractérisé en ce que** la tige (83) est constituée par des parties individuelles inflexibles qui sont reliées de manière articulée les unes aux autres dans sa direction longitudinale à des emplacements espacés les uns des autres.

5. Procédé de nettoyage de la cavité d'un lumen (82) d'un objet médical compostant un dispositif selon l'une des revendications 1 à 4, **caractérisé en ce que** dans le lumen (82) de l'objet (81), un flux de liquide de transmission dirigé à l'encontre de la direction d'avancement de la tige est additionnellement engendré pour faire sortir du lumen (82) les particules détachées par les jets (84').

6. Procédé selon la revendication 5 ou 6, **caractérisé en ce que** la tige (85) est remuée pendant l'opération de nettoyage.
